# EUROPEAN PATENT SPECIFICATION

(11) **EP 0 404 421 B1**
(45) Date of publication and mention of the grant of the patent: **24.08.1994**
(21) Application number: 90306384.0
(22) Date of filing: 12.06.1990
(51) Int. Cl.: C07C 15/24, C07C 7/14, B01D 9/00

(54) **Method of separating and purifying beta-monoisopropyl-naphthalene**
Verfahren zur Abtrennung und Reinigung von beta-Monoisopropylnaphthalen
Procédé pour la séparation et l'épuration de béta-monoisopropylnaphtalène

(30) Priority: 22.06.1989 JP 158413/89
(43) Date of publication of application: 27.12.1990
(73) Proprietor: KUREHA KAGAKU KOGYO KABUSHIKI KAISHA, Chuo-ku Tokyo 103 (JP)
(72) Inventor: Takahashi, Nobuyuki, Iwaki-shi, Fukushima-ken 974 (JP); Takayama, Hajime, Iwaki-shi, Fukushima-ken 974 (JP); Matsuoka, Takeshi, Iwaki-shi, Fukushima-ken 974 (JP); Mukaida, Tamito, Suita-shi, Osaka 565 (JP)
(74) Representative: Jones, Helen Marjorie Meredith

(56) References cited:
- DE-C- 920 243
- US-A- 2 777 889
- US-A- 3 886 223
- CHEMICAL ABSTRACTS, vol. 111, no. 9, 28 August 1989, page 721, abstract no. 77673, Columbus, Ohio, US; & JP-A-0161436

## Description

This invention relates to a method of separating and purifying β-monoisopropylnaphthalene in a high purity by a crystallization method from a monoisopropylnaphthalene (hereinafter abbreviated as "MIPN") fraction obtained by the isopropylation and transisopropylation of naphthalene.

### BACKGROUND OF THE INVENTION

β-MIPN is used as a raw material for preparing β-naphthol and β-isopropenylnaphthalene. β-Naphthol is used as a raw material for preparing raw materials for medicines, organic synthesis and beneficiation agents, while β-isopropenylnaphthalene is used as a raw material for preparing a variety of macromolecular materials. However, these compounds are required to have a high purity in order to use in such applications. It is therefore desirable that β-MIPN, a precursor thereof, is also purified in a purity as high as at least 99%.

By the way, β-MIPN is generally prepared by isopropylating naphthalene. However, an MIPN fraction obtained by the isopropylation of naphthalene is a mixture containing α-MIPN and β-MIPN.

For example, separation by distillation is thought as a method of separating and purifying β-MIPN from this MIPN fraction. However, since the boiling points of α-MIPN and β-MIPN are close to each other as 267.4°C and 268.9°C respectively, it is substantially impossible to industrially separate and purify β-MIPN in a high purity by the distillation.

On the other hand, the melting points of α-MIPN and β-MIPN are -15.7°C and +15.1°C respectively, and a difference of about 30°C hence exists between both of them. It is therefore considered that the separation and purification by a crystallization method is allowed. However, when a mixture containing α-MIPN and β-MIPN is cooled in a crystallization vessel to crystallize and separate β-MIPN having a higher melting point, crystals to be deposited become crystallites because the generation rate of crystal nuclei is great, the liquid viscosity of the mixture is relatively high and the speed of crystal growth is small. It is hence difficult to separate the crystals from the mother liquor. In addition, since the crystals grow out of the wall surface of the crystallization vessel, the maintenance of a uniform slurry state becomes impossible. It is hence difficult to stir the interior of the crystallization vessel. Therefore, it has been considered heretofore that difficulties are encountered on the separation and purification of β-MIPN in a purity as high as at least 99% by the crystallization method from the MIPN fraction.

Hence, it has been proposed to crystallize β-MIPN by using lower alcohol as a solvent (Japanese Patent Publication No. 23406/1981). Although high-purity β-MIPN can be obtained according to this solvent crystallization method, the method is uneconomical as an industrial method because it requires a recovery process for the solvent and a particular equipment therefor.

### OBJECTS AND SUMMARY OF THE INVENTION

It is an object of the present invention to provide a method of efficiently separating and purifying high-purity β-MIPN from an MIPN fraction by a crystallization method without using any solvents.

The present inventors have carried out an extensive investigation with a view toward overcoming the above-described problems of the prior art. As a result, it has been found that upon the separation and purification of β-MIPN by a crystallization method from an MIPN fraction obtained by the isopropylation and transisopropylation of naphthalene, when a fraction containing at least 85 wt.% of β-MIPN and at most 0.2 wt.% of unreacted naphthalene is selected as the MIPN fraction, a scraper-equipped, externally-cooled mixing tank type crystallization vessel is used as a crystallization vessel, and the crystallization is conducted under selected process conditions, high-purity β-MIPN having a purity as high as at least 99% can be separated with ease.

It has also be found that when a liquid component obtained by solid-liquid separation in a crystallization process, or the like is recycled for reuse as at least one part of the MIPN fraction, high-purity β-MIPN can be obtained continuously with good efficiency.

The present invention has been led to completion on the basis of these findings.

According to the present invention, there is thus provided a method for the separation and purification of β-MIPN, characterized in that an MIPN fraction containing at least 85 wt.% of β-MIPN and at most 0.2 wt.% of naphthalene is derived from products formed by the isopropylation and transisopropylation of naphthalene, and the MIPN fraction is fed to a scraper-equipped, externally-cooled mixing tank type crystallization vessel to subject it to crystallization under the following conditions:

| | |
|---|---|
| difference in temperature between the internal and external wall surfaces of the crystallization vessel: | 1-15°C |
| temperature inside the crystallization vessel: | 5-12°C |
| peripheral speed of the scraper: | 0.1-4 m/sec |
| residence time: | 2-20 hours |

According to this invention, it is possible to separate β-MIPN as a crystal component having a purity of at least 99% in an amount of 20-40 wt.% of the β-MIPN feed.

### DETAILED DESCRIPTION OF THE INVENTION

Features of the present invention will hereinafter be described in detail.

In this invention, an MIPN fraction containing β-MIPN in an amount of at least 85 wt.%, generally, in a range of 85-93 wt.% and naphthalene in an amount of at most 0.2 wt.%, preferably at most 0.1 wt.% is derived from products formed by the isopropylation and transisopropylation of naphthalene to use it in a crystallization process.

The MIPN fraction having such a composition can be obtained by rectifying products, which have been obtained by isopropylating naphthalene to a degree of isopropylation of 1 and further transisopropylating the thus-isopropylated naphthalene at 280-300°C, under selected conditions.

In this invention, a residual liquid component from which β-MIPN crystals formed by the crystallization have been removed by solid-liquid separation may be either caused to flow back as at lease one part of the MIPN fraction as is so long as the liquid component contains at least 85 wt.% of β-MIPN and the content of naphthalene is not more than 0.2 wt.%, or provided again for the transisopropylation process, thereby recycling it for use as at least one part of the MIPN fraction which has been prepared in the above-described composition range.

In this invention, a crude β-MIPN crystal component having a purity of 90-98%, preferably 95-98% and obtained by feeding the residual liquid component from which β-MIPN has been separated as a crystal component to a second scraper-equipped, externally-cooled mixing tank type crystallization vessel and enhancing the recovery efficiency, for example, by making the difference in temperature between both wall surfaces greater, may be used as at least one part of the above MIPN fraction so as to separate and purify β-MIPN with better efficiency.

Accordingly, the MIPN fraction fed to the crystallization vessel in this invention may contain the above-described flowing back component and the like.

If the content of naphthalene in the MIPN fraction fed to the crystallization vessel should exceed 0.2 wt.%, the deposition of crystallites will become increased, whereby difficulties are encountered on the separation of crystals from the mother liquor. It is hence impossible to obtain β-MIPN having a purity as high as at least 99%.

On the other hand, if the content of β-MIPN in the MIPN fraction should be less than 85 wt.%, the inclusion of the mother liquor into crystals will become greater, whereby difficulties are encountered on the separation of the mother liquor. It is also difficult to obtain high-purity β-MIPN.

By the way, crystals of an intended product are deposited by cooling a solution to be treated to a predetermined temperature range in a crystallization operation. However, when the liquid temperature of the MIPN fraction is lowered rapidly, the number of crystal nuclei generated is increased, whereby the crystal component is deposited as crystallites. This tendency becomes noticeable as the content of naphthalene in the MIPN fraction is higher. If the temperature upon the crystallization is high on the other hand, the recovery of the crystals becomes lowered.

Thus, in order to obtain β-MIPN in a purity of at least 99% by the crystallization method, it is important to use, as a solution to be treated, an MIPN fraction prepared so as to contain at least 85 wt.% of β-MIPN and at most 0.2 wt.% of naphthalene and at the same time, to select crystallization conditions.

In this invention, a scraper-equipped, externally-cooled mixing tank type crystallization vessel is used, and an operation temperature of the crystallization vessel, a difference in temperature between the internal and external wall surfaces of the crystallization vessel and a peripheral speed of the scraper are controlled respectively to 5-12°C, preferably 7-10°C, to 1-15°C, preferably 2-5°C and to 0.1-4 m/sec, preferably 0.5-2 m/sec. When operated under such crystallization conditions, it is possible to separate and purify β-MIPN as crystals having a purity of at least 99% in an amount of 20-40 wt.% of the β-MIPN feed in a residence time of 2-20 hours, preferably 4-12 hours.

Any temperatures inside the crystallization vessel lower than 5°C or any peripheral speeds of the scraper faster than 4 m/sec will result in deposition of too fine crystals, whereby difficulties are encountered on their separation from the mother liquor. It is hence impossible to obtain high-purity β-MIPN. Any temperatures inside the crystallization vessel higher than 12°C or any differences in temperature between both wall surfaces smaller than 1°C will result in lowered recovery. Any peripheral speeds of the scraper slower than 0.1 m/sec or any differences in temperature between both wall surfaces greater than 15°C will result in a phenomenon that the crystal layer on the wall surface is rotated together with the scraper rather than scraped off. Therefore, resistance to stirring will become greater and the coefficient of heat transfer of the wall surface will be lowered correspondingly. If the residence time of the MIPN fraction in the crystallization vessel should be too short, a low-purity β-MIPN will be obtained. On the other hand, any residence time too long will be not efficient.

The residual liquid component from which the crystal component has been removed by solid-liquid separation may be caused to flow back again to the crystallization vessel so long as it contains at least 85 wt.% of β-MIPN and the content of naphthalene is not more than 0.2 wt.%. The liquid component may also be provided again for the transisopropylation process to reprepare for recycling as an MIPN fraction having the above-described composition. In this manner, continuous crystallization can be performed.

As another embodiment of this invention, there is a method wherein two scraper-equipped, externally-cooled mixing type crystallization vessels are connected to each other to conduct a crystallization process, thereby enhancing the recovery of β-MIPN. Described specifically, an MIPN fraction obtained by the isopropylation and transisopropylation of naphthalene is fed to the first crystallization vessel to subject it to crystallization under the above-described conditions, thereby β-MIPN crystals having a purity of at least 99%. A residual liquid component obtained by solid-liquid separation is then fed to the second crystallization vessel to subject it to crystallization.

In the second crystallization vessel, crude β-MIPN crystals having a purity of at least 90%, generally 90-98% is obtained, for example, by making the difference in temperature between both wall surfaces greater than that in the first crystallization vessel in order to enhance the recovery of β-MIPN. The thus-obtained crude β-MIPN crystals are fed to the first crystallization vessel in combination with the MIPN fraction. If the purity of the crude β-MIPN is lower than 90%, the recovery efficiency is not very increased even when the second crystallization vessel is connected. It is preferable to operate in the second crystallization vessel so as to obtain crude β-MIPN having a purity of 95-98%. A liquid component from the second crystallization vessel, which has been obtained by solid-liquid separation, is returned to the transisopropylation process to recycle as an MIPN fraction.

### ADVANTAGES OF THE INVENTION

According to the present invention, it has been possible to provide β-MIPN having a purity of at least 99% in accordance with the crystallization method by using an MIPN fraction having a specific composition and selecting the specific crystallization process conditions described above in a residence time of 2-20 hours without using any organic solvents required heretofore.

### EMBODIMENTS OF THE INVENTION

### [Examples]

The present invention hereinafter be described more specifically by the following examples and comparative examples.

### Example 1:

A scraper-equipped, externally-cooled mixing tank type crystallization vessel (internal volume: 320 liters) was fed with an MIPN fraction derived from products formed by the isopropylation and transisopropylation of naphthalene and containing 90 wt.% of β-MIPN and less than 0.1 wt.% of naphthalene at a rate of 23.1 kg/hr. Crystallization was conducted under the following conditions:

| | |
|---|---|
| difference in temperature between the internal and external wall surfaces of the crystallization vessel: | 4°C |
| temperature inside the crystallization vessel: | 8°C |
| peripheral speed of the scraper: | 1 m/sec |
| residence time: | 10 hours |

and solid-liquid separation was then carried out. As a result, β-MIPN crystals having a purity of 99.3% were obtained at a rate of 7.3 kg/hr (about 35 wt.% of the β-MIPN feed). A residual liquid component containing 85.7 wt.% of β-MIPN was returned to the transisopropylation process.

### Example 2:

Crystallization was conducted in the same manner as in Example 1 except that an MIPN fraction whose β-MIPN content was 89.9 wt.% and whose naphthalene content was 0.1 wt.% was used as the MIPN fraction fed to the crystallization vessel. As a result, β-MIPN crystals having a purity of 99.1% were obtained at a rate of 7.3 kg/hr (about 35 wt.% of the β-MIPN feed). A residual liquid component containing 85.6 wt.% of β-MIPN was returned to the transisopropylation process.

### Comparative Example 1:

Crystallization was conducted in the same manner as in Example 1 except that an MIPN fraction whose β-MIPN content was 89.6 wt.% and whose naphthalene content was 0.5 wt.% was used as the MIPN fraction fed to the crystallization vessel. The purity of the resulting β-MIPN was found to be 98.8%.

### Comparative Example 2:

Crystallization was conducted in the same manner as in Example 1 except that an MIPN fraction whose β-MIPN content was 89.1 wt.% and whose naphthalene content was 1.0 wt.% was used as the MIPN fraction fed to the crystallization vessel. The purity of the resulting β-MIPN was found to be 98.6%.

### Comparative Example 3:

The same crystallization vessel as that used in Example 1 was fed with an MIPN fraction whose β-MIPN content was 90 wt.% and whose naphthalene content was less than 0.1 wt.% at a rate of 304.0 kg/hr. Crystallization was conducted in a residence time as short as 1 hour while controlling a difference in temperature between both wall surfaces of the crystallization vessel, a temperature inside the crystallization vessel and a peripheral speed of the scraper to 14°C, 8°C and 1 m/sec respectively. As a result, β-MIPN crystals having a purity of 96.3% were obtained at a rate of 20.8 kg/hr.

### Comparative Example 4:

The same crystallization vessel as that used in Example 1 was fed with an MIPN fraction whose β-MIPN content was 90 wt.% and whose naphthalene content was less than 0.1 wt.% at a rate of 50.7 kg/hr. Crystallization was conducted while enlarging a difference in temperature between both wall surfaces of the crystallization vessel to 24°C and controlling a temperature inside the crystallization vessel, a peripheral speed of the scraper and a residence time to 7°C, 1 m/sec and 6 hours respectively. As a result, β-MIPN crystals having a purity of 96.7% were obtained at a rate of 14.1 kg/hr.

### Example 3:

The liquid component obtained by the solid-liquid separation in Example 1 and containing 85.7 wt.% of β-MIPN was not returned to the transisopropylation process, but fed to a crystallization vessel (the second crystallization vessel) connected to the crystallization vessel (the first crystallization vessel) used in Example 1 and having the same type as the first crystallization vessel at a rate of 21.4 kg/hr. The remainder was returned to the first crystallization vessel.

In the second crystallization vessel, crystallization was conducted under the following conditions:

| | |
|---|---|
| difference in temperature between both wall surfaces of the crystallization vessel: | 18°C |
| temperature inside the crystallization vessel: | 7°C |
| peripheral speed of the scraper: | 1 m/sec |
| residence time: | 14 hours |

and solid-liquid separation was then carried out. As a result, crystals having a purity of 97.8% were obtained at a rate of 5.7 kg/hr (about 30 wt.% of the β-MIPN feed). A liquid component obtained by the solid-liquid separation was returned to the transisopropylation process.

Low-purity crude β-MIPN crystals obtained in the second crystallization vessel were melted and combined with the MIPN fraction obtained by the isopropylation and transisopropylation of naphthalene and a part of the flowing back component from the first crystallization vessel to give a composition that the β-MIPN content was 90.0 wt.% and the naphthalene content was less than 0.1 wt.%, followed by feeding to the first crystallization vessel at a rate of 23.1 kg/hr.

In the first crystallization vessel, crystallization was conducted under the following conditions:

| | |
|---|---|
| difference in temperature between both wall surfaces of the crystallization vessel: | 4°C |
| temperature inside the crystallization vessel: | 8°C |
| peripheral speed of the scraper: | 1 m/sec |
| residence time: | 10 hours |

and solid-liquid separation was then carried out. As a result, β-MIPN crystals having a purity of 99.3% were obtained at a rate of 7.3 kg/hr (about 35 wt.% of the β-MIPN feed).

## Claims

1. A method for the separation and purification of β-monoisopropylnaphthalene, characterized in that a monoisopropylnaphthalene fraction containing at least 85 wt.% of β-monoisopropylnaphthalene and at most 0.2 wt.% of naphthalene is derived from products formed by the isopropylation and transisopropylation of naphthalene, and the monoisopropylnaphthalene fraction is fed to a scraper-equipped, externally-cooled mixing tank type crystallization vessel to subject it to crystallization under the following conditions:
| | |
|---|---|
| difference in temperature between the internal and external wall surfaces of the crystallization vessel: | 1-15°C |
| temperature inside the crystallization vessel: | 5-12°C |
| peripheral speed of the scraper: | 0.1-4 m/sec |
| residence time: | 2-20 hours |

2. The method as claimed in Claim 1, wherein a residual liquid component from which β-monoisopropylnaphthalene has been separated as the crystal component is reused as at least one part of the monoisopropylnaphthalene fraction as it is or after providing for the transisopropylation process.

3. The method as claimed in Claim 1 or 2, wherein a residual liquid component from which β-monoisopropylnaphthalene has been separated as the crystal component is fed to a second scraper-equipped, externally-cooled mixing tank type crystallization vessel and the resulting crude β-monoisopropylnaphthalene crystals having a purity of 90-98% is melted to use it as at least one part of the monoisopropylnaphthalene fraction.

## Patentansprüche

1. Verfahren zur Abtrennung und Reinigung von β-Monoisopropylnaphthalin, dadurch gekennzeichnet, daß eine Monoisopropylnaphthalin-Fraktion, die mindestens 85 Gew.-% β-Monoisopropylnaphthalin und höchstens 0,2 Gew.-% Naphthalin enthält, abgeleitet wird von Produkten, die bei der Isopropylierung und Transisopropylierung von Naphthalin gebildet werden, und die Monoisopropylnaphthalin-Fraktion in ein mit einem Schaber ausgerüstetes, extern gekühltes Kristallisationsgefäß vom Mischtank-Typ eingeleitet wird, um sie der Kristallisation unter folgenden Bedingungen zu unterwerfen:
| | |
|---|---|
| Temperaturdifferenz zwischen den inneren und äußeren Wandoberflächen des Kristallisationsgefäßes | 1-15°C |
| Innentemperatur des Kristallisationsgefäßes | 5-12°C |
| Umfangsgeschwindigkeit des Schabers | 0,1-4 m/s |
| Verweilzeit | 2-20 h |

2. Verfahren nach Anspruch 1, worin eine Rest-Flüssigkeitskomponente, aus der β-Monoisopropylnaphthalin als Kristallkomponente abgetrennt worden ist, wiederverwendet wird als zumindest ein Teil der Monoisopropylnaphthalin-Fraktion, entweder als solche oder nach erfolgter Transisopropylierung.

3. Verfahren nach Anspruch 1 oder 2, worin eine Rest-Flüssigkeitskomponente, aus der β-Monoisopropylnaphthalin als Kristallkomponente abgetrennt worden ist, in ein zweites mit einem Schaber ausgerüstetes, extern gekühltes Kristallisationsgefäß vom Mischtank-Typ eingeleitet wird, und die erhaltenen rohen β-Monoisopropylnaphthalin-Kristalle mit einer Reinheit von 90-98% geschmolzen werden, um sie als zumindest ein Teil der Monoisopropylnaphthalin-Fraktion zu verwenden.

## Revendications

1. Procédé pour la séparation et la purification de β-monoisopropylnaphtalène, caractérisé en ce qu'une fraction de monoisoproprylnaphtalène contenant au moins 85 % en poids de β-monoisopropylnaphtalène et au plus 0,2 % en poids de naphtalène est dérivée des produits formés par isopropylation et transisopropylation du naphtalène, la fraction de monoisopropylnaphtalène étant fournie à une enceinte de cristallisation du type réservoir de mélange, refroidie de l'extérieur, équipée d'un racloir, pour la soumettre à cristallisation dans les conditions suivantes :
| | |
|---|---|
| différence de température entre les surfaces de parois interne et externe de l'enceinte de cristallisation | 1- 15°C |
| température à l'intérieur de l'enceinte de cristallisation | 5- 12°C |
| vitesse périphérique du racloir | 0,1-4 m/sec |
| temps de séjour | 2 - 20 heures |

2. Procédé selon la revendication 1, dans lequel un constituant liquide résiduel , duquel le β-monoisopropylnaphtalène a été séparé comme composé cristallin, est réutilisé tel quel comme au moins une partie de la fraction de monoisopropylnaphtalène ou après fourniture au processus de transisopropylation.

3. Procédé selon la revendication 1 ou 2, dans lequel un composé résiduel liquide, duquel le β-monoisopropylnaphtalène a été séparé comme composé cristallin, est fourni à une seconde enceinte de cristallisation du type à réservoir de mélange refroidie de l'extérieur, équipée d'un racloir et les cristaux bruts de β-monoisopropylnaphtalène obtenus ayant une pureté de 90-98 % sont fondus afin de les utiliser comme au moins une partie de la fraction de monoisopropylnaphtalène.
